# EUROPEAN PATENT APPLICATION

(11) **EP 4 763 985 A1**
(43) Date of publication of application: **24.06.2026**
(21) Application number: 24307237.8
(22) Date of filing: 20.12.2024
(51) Int. Cl.: C12N 15/113, A61K 31/7088, A61P 31/14

(54) **ANTISENSE OLIGONUCLEOTIDES AS THERAPEUTIC AGENTS AGAINST SARS COV-2**

(71) Applicant: Centre National de la Recherche Scientifique, 75016 Paris (FR); Institut Pasteur, 75015 Paris (FR); Ecole Normale Supérieure Paris-Saclay, 91190 Gif-sur-Yvette (FR)
(72) Inventor: YOSHIZAWA, Satoko, 78460 Chevreuse (FR); FOURMY, Dominique, 78460 Chevreuse (FR); BOULLE, Mikael, 56220 Rochefort-en-Terre (FR); AGOU, Fabrice, 75012 Paris (FR); FACCHINETTI, Lucas, 77114 Noyen-sur-Seine (FR)
(74) Representative: Novagraaf Technologies

(57) **Abstract**

The present invention concerns the creation of antisense oligonucleotides (ASOs) with no cytotoxicity that are very active as therapeutic agents in knocking down the replication of SARS-CoV-2 in human cells with pan activity against all known past and current variants.

## Description

### Technical field of the invention

The present invention concerns the creation of antisense oligonucleotides (ASOs) with no cytotoxicity that are very active as therapeutic agents in knocking down the replication of SARS-CoV-2 in human cells with pan-antiviral activity against all known past and current variants.

The main application is the treatment of patients infected with SARS-CoV-2.

### State of the art

The coronavirus disease 2019 (COVID-19) pandemic is ongoing for now several years and as of now caused more than 7 million death worldwide (https://data.who.int/dashboards/covid19/deaths, https://www.who.int/news/item/05-05-2023-statement-on-the-fifteenth-meeting-of-the-international-health-regulations-(2005)-emergency-committee-regarding-the-coronavirus-disease-(covid-19)-pandemic). WHO also released a more complete study: "New estimates from the World Health Organization (WHO) show that the full death toll associated directly or indirectly with the COVID-19 pandemic (described as "excess mortality") between 1 January 2020 and 31 December 2021 was approximately 14.9 million (range 13.3 million to 16.6 million).(https://www.who.int/news/item/05-05-2022-14.9-million-excess-deaths-were-associated-with-the-covid-19-pandemic-in-2020-and-2021)".

Vaccines are being developed to help control the pandemic, but novel viral variants constantly emerge with increased infectivity and the potential to evade vaccine-induced immunity, thus threating the efficacy of the current vaccination campaigns and posing significant challenges in controlling the COVID-19 pandemic.

Gapmer ASOs are characterized by a central segment of DNA flanked by modified nucleotides, enabling them to specifically bind to target RNA sequences and recruit the intracellular RNase H1 that specifically cleaves the formed DNA/RNA heteroduplexes [1,2]. This approach is a very attractive therapeutic strategy when combined with efficient cellular delivery strategies and several drugs have gained approval [3-5]. As of September 2024, nineteen oligonucleotide drugs have received approval from the FDA [6-8]. But to date there is no FDA approved oligonucleotide drug targeting SARS-CoV-2 or other respiratory viruses.

The number of groups worldwide developing gapmer ASOs (substrate of RNAse H) or mixmer ASOs (interspersed combination of LNA and DNA nucleotides), specifically targeting SARS-CoV-2 RNA for knocking down viral replication has recently drastically increased however, their activity remains unsatisfactory :
The group of Rhiju Das (Department of Biochemistry Stanford University, Stanford, CA, USA) recently reported the cryo-EM structure of the frameshift stimulation element (FSE) domain and the structure-based design of ASOs [9]. This structure might not recapitulate the *in vivo* structure as it was done *in vitro* and not in the context of the full SARS-CoV-2 RNA genome. The LNA ASOs were designed with the goal of disrupting the FSE structure to inhibit -1 programmed ribosomal frameshifting (- 1 PRF). The activity of the designed ASOs remained weak, i.e. required > 100 nM to partially prevent SARS-CoV-2 replication in A549-ACE2 cells.

The groups of Silvi Rouskin (Whitehead Institute for Biomedical Research, Cambridge, MA 02142, USA), and Anders M. Näär (Department of Nutritional Sciences & Toxicology, University of California, Berkeley, CA 94720, USA) performed a massive study using more than 100 LNA mixmer or gapmer ASOs targeting SARS-CoV-2 RNA [10]. They designed 42 mixmers to target the FSE region. Indeed some of the mixmers target the 13350-13600 region (numbering of Wuhan sequence NC_045512.2). They did not find this region to be a potentially very attractive target. Instead the study revealed that "LNA ASOs targeting the 5' leader region of SARS-CoV-2 were particularly effective in suppressing viral RNA levels in infected cells". They focused therefore on their best mixmer that targets the 5' leader region for development of an intranasal therapeutic ASO. Facing the difficulty in generating ASOs with pan-antiviral activity against all circulating and future SARS-CoV-2 variants, the authors mention in their discussion the possibility to use a combination of several ASOs in order to have a treatment resistant to the apparition of variants "Combinatorial LNA ASO cocktails targeting multiple essential genomic regions of viruses may further increase the efficacy of LNA ASOs as therapeutic candidates to overcome viral evasion mutations". However, such a strategy might be heavy to implement for instance with increasing the chance of cytotoxicity effects.

The group of Anna Marie Pyle (Department of Molecular, Cellular, and Developmental Biology, Yale University, New Haven, CT 06511, USA) designed LNA ASOs targeting three different regions along the viral RNA with the same objective of disrupting the RNA structure and failed to obtain a good activity [11].

The group of Feng Guo (Department of Biological Chemistry, David Geffen School of Medicine, University of California, Los Angeles, CA 90095, U.S.A.) is developing ASOs that target SARS-CoV-2 RNA with the objective of making sure ASO binding is compatible with target structures in three-dimensional (3D) space by employing structural design templates [12]. The design of ASOs makes use of a phosphorodiamidate morpholino oligomer (PMO) skeleton. They targeted a unique sequence within the FSE region (13531-13551 region, numbering of Wuhan sequence NC_045512.2) but ASOs activity remains weak (EC₅₀ > 2 µM).

The group of Ben Luisi (Department of Biochemistry, University of Cambridge, Cambridge, United Kingdom) developed ASOs targeting the stem-loop 2 motif (s2m) in the 3' untranslated region (UTR) of the RNA [13]. The designed ASOs were gapmers but because the target site is a highly structured stem-loop, ASOs failed to have a good activity (> 0.5 µM).

The groups of Jonathan Z. Sexton (University of Michigan, USA) and Arul M. Chinnaiyan (University of Michigan, USA) tested ASOs including 48 ASOs that are uniformly 2'-O-methoxyethyl (2'MOE)-modified and 132 Gapmer ASOs containing 2' constrained ethyl (2'cEt) modifications [14]. These ASOs span throughout entire SARS-CoV-2 genome. However, none of these ASOs exhibited strong activity requiring more than 1µM of ASO to inhibit 50% of viral infection.

Recently group of Koichi Watashi (National Institute of Infectious Disease, Japan) reported their work on 292 anti-SARS-CoV-2 gapmer ASOs [15]. Their screening identified ASO#41 targeting coding region of M^{pro} (Nsp5) protease as the most active ASO. ASO#41 showed an activity slightly worse than the best mixmer of the study of Zhu et al., [10] and on three variants of SARS-CoV-2. They also tested ASO#41 sequence and the neighboring sequence with various backbone modifications such as amido-bridged nucleic acid (AmNA), 2'-O, 4'-C-ethylene-bridged nucleic acid, 2'MOE modified ASO or phosphoryl guanidine-containing backbone linkages, however their anti-SARS-CoV-2 activities remained moderate (IC₅₀ > 23 nM) and without complete inhibition at high concentration (200 nM). The authors did not test pan-activity for the most active ASO.

Another type of ASOs has been tested against SARS-CoV-2 and HCoV-OC43 viruses [16]. These are modified peptide nucleic acids called OPNAs where a cationic lipid moiety was introduced into nucleobase to improve cell permeability. They aligned SARS-CoV-2 and HCoV-OC43 sequences and designed 83 sense or antisense OPNAs targeting conserved sequences. In particular, the authors targeted a specific site of the FSE region with a series of OPNAs of different length and modifications for mitigated activity against the tested HCoV-OC43 virus (55% of inhibition at 1µM OPNA). This series of OPNAs did not show superior activity compared to other targeted sites. The authors then used a combination of two, three or four OPNAs targeting different sites in attempts to obtained better results. This strategy did not drastically improve the results on HCoV-OC43 and SARS-CoV-2. Using 2.5 µM of each OPNAs, viral RNA was reduced only by 50 %. Another strategy has been the design of siRNAs or CRISPR RNAs targeting SARS-CoV-2 RNA for knocking down viral replication however, without completely convincing results :
The groups of Yuanchao Xue (Key Laboratory of RNA Biology, Institute of Biophysics, Chinese Academy of Sciences, Beijing, China) and Jianwei Wang (National Health Commission of the People's Republic of China Key Laboratory of Systems Biology of Pathogens and Christophe Mérieux Laboratory, Institute of Pathogen Biology, Chinese Academy of Medical Sciences & Peking Union Medical College, Beijing, China.) studied the structure of the viral RNA inside virions to facilitate structure-based drug development such as efficient siRNAs [17]. They designed 6 siRNA against conserved and single-stranded regions and four of them had activity in limiting the number of viral RNA copies in the supernatant of infected Vero cells or inside Vero cells. All 6 siRNA target outside the FSE.

The groups of Nigel McMillan (Menzies Health Institute Queensland, School of Medical Science Griffith University, Gold Coast Campus, QLD 4222, Australia) and Kevin Morris (Menzies Health Institute Queensland, School of Medical Science Griffith University, Gold Coast Campus, QLD 4222, Australia; Center for Gene Therapy, Hematological Malignancy and Stem Cell Transplantation Institute at the City of Hope and City of Hope Beckman Research Institute, 1500 E. Duarte Road, Duarte, CA 91010, USA) developed siRNAs delivered using a novel lipid nanoparticle (LNP) system [18]. They tested the activity of 17 siRNAs. sLNP-siRNA were administered intravenously. The same group recently applied this strategy for targeting variants of concern up to 2023 [19], however in this work they did not reveal the sequences of the siRNAs used.

The group of Lei Qi (Department of Bioengineering, Stanford University, Stanford, CA 94305, USA) designed and screened CRISPR RNAs (crRNAs) targeting conserved viral regions and identified functional crRNAs targeting SARS-CoV-2 [20]. Their objective was to create Pan-coronavirus crRNAs capable of inactivating multiple types of viruses. But the authors cite a strong limitation: "the biggest barrier to deploying PAC-MAN clinically is the development of effective and safe *in vivo* delivery methods".

Therefore, there is a critical need for novel therapeutic strategies able to target circulating and future SARS-CoV-2 variants. Despite, the abundant literature, summarized above, containing a very high number of ASOs generated that target SARS-CoV-2 including some against the FSE region targeted by the inventors a very efficient ASO is still missing. Therefore, it is difficult even for a person skilled in the art to explore possiblities to create ASOs targeting the FSE region with very high efficiency. The results also highlight the difficulties in making ASOs with very efficient pan-antiviral activity against circulating and future SARS-CoV-2 variants.

### Description of the invention

The technical problem to solve is to efficiently inhibit replication of SARS-CoV-2 in infected cells with a strategy that would be resistant to the apparition of variants.

To this aim the inventors provide a solution through the development of a gapmer antisense oligonucleotide (ASO) therapeutical strategy as a promising approach for targeting a specific domain of SARS-CoV-2 genomic RNA that leads to the inhibition of viral replication.

Several criteria need to be fulfilled, especially: i) ultra-conservation of target sequences to confer resistance to variants and hence universality of the therapeutic agent, ii) the ASOs should not be cytotoxic to human cells, iii) binding of the ASOs to the target sequence should be highly efficient for maximal inactivation of SARS-CoV-2 RNA. Because ASOs rely on base-pairing to a nucleotide sequence composed of about 20 consecutive bases, the occurrence of a single point mutation within the target RNA is less likely to confer resistance to the treatment.

SARS-CoV-2 uses programmed ribosomal frameshifting (PRF) to enable synthesis of the viral RNA-dependent RNA polymerase and downstream proteins. The inventors found an ultra-conserved region, encompassing the slippery sequence, the linker of the frameshifting RNA element (FSE) domain as well as flanking regions. The inventors thus targeted this region using specifically designed gapmer antisense oligonucleotides (ASOs) to allow, with high efficiency, inhibiting viral replication against all SARS-CoV2 variants and offering a new therapeutic approach.

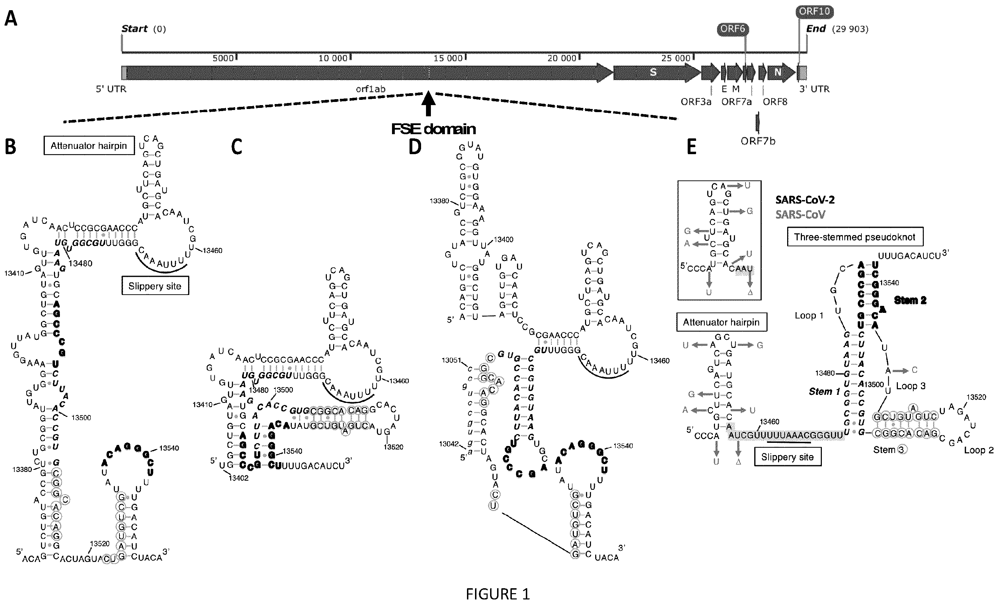

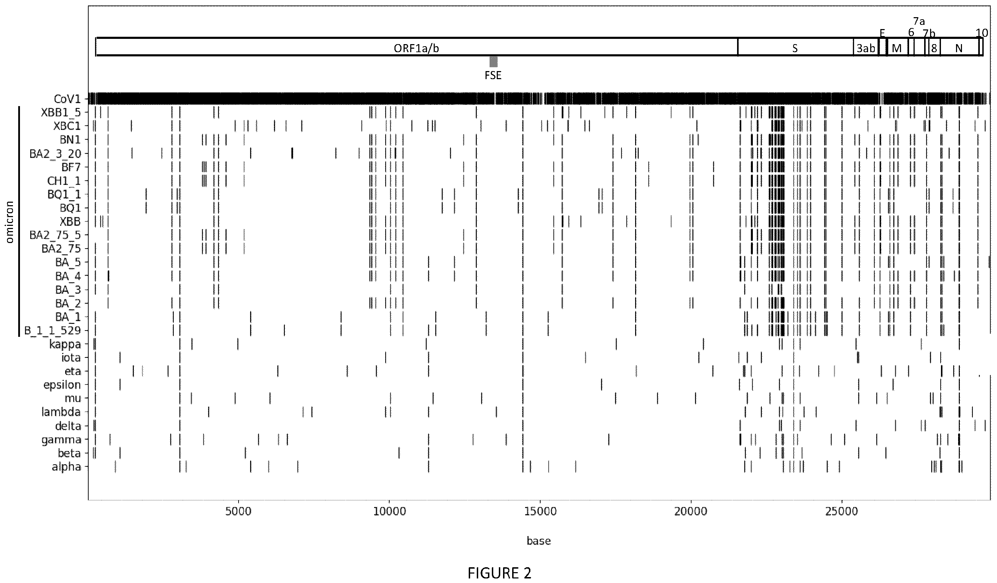

Through a careful design and microwalk approach the inventors thus generated several therapeutic gapmer ASOs to target a specific region of SARS-CoV-2 RNA known as the -1 frameshiftting element (-1 FSE) [21,22], in particular towards the slippery sequence of this -1 FSE region, with no cytotoxicity, that knock down viral replication in human cells at a very strong activity at a few nM concentration, validating the ASO-based therapy against SARS-CoV-2. This targeted region is an RNA domain that can adopt multiple structures (Figure 1) [11,21,23,24,25]. This FSE region is highly conserved across all SARS-CoV-2 variants (Figure 2). The FSE is crucial for inducing a frameshift during the translation of viral genomic RNA, which is essential for the complete synthesis of the first viral polyprotein ORF1a/1b in infected cells [21]. The conservation of this region among all SARS-CoV-2 variants, from the original Wuhan strain to current variants, underscores its potential as a therapeutic target with a pan-antiviral activity against all past, present, and future SARS-CoV-2 variants.

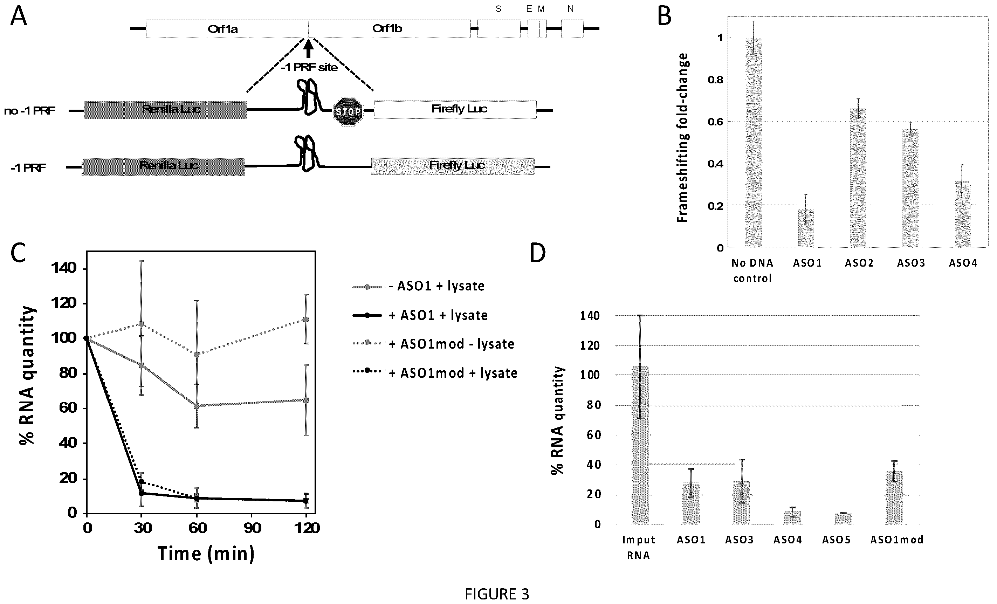

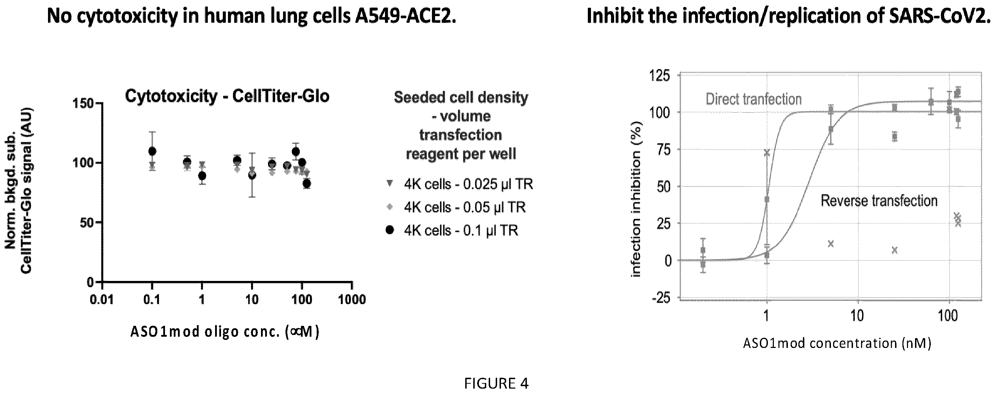

The inventors first showed that the FSE domain is an excellent target for an ASO based therapeutic strategy and identified ASO4 and ASO5 (unmodified ASOs) that displayed remarkable activity by targeting the slippery sequence of the -1 FSE domain. These two ASOs originated from ASO1 as the original ASO (Figure 3 B,C,D). To be noted ASO1mod, a modified version of ASO1 (a gapmer containing 2'OMe modification combined with phosphorothiotate linkages; see table 4) displayed a remarkable IC50 around 1 to 5 nM (Figure 4). The inventors next extended the study to more advance ASOs containing modifications on multiple SARS-CoV-2 variants for pan-antiviral activity identification. To assess therapeutic efficacy and safety, they infected human lung cell lines with the authentic B.1.617.2 (Delta) SARS-CoV-2 variant under BSL-3 conditions, using human cells pre-transfected with the designed gapmer ASOs. They then determined the potential inhibition effect of the ASOs on virus replication by measuring viral RNA levels via RT-qPCR post-infection and evaluated cytotoxicity using a luminescence cell viability assay to determine their therapeutic indices (CC₅₀/IC₅₀). They showed that two gapmers, ASO6 and ASO7, exhibited potent antiviral activity with nanomolar IC₅₀ inhibition potencies against a broad range of SARS-CoV-2 variants, including Beta, Delta, BA.2.86, XBB.1.5, and JN.1, validating their therapeutic strategy.

An object of the present invention concerns a gapmer antisense oligonucleotide (ASO) targeting SARS-CoV-2 viral RNAs, characterized in that it comprises phosphorothioate linkages and a central sequence flanked by modified nucleotides on the 5' and 3' sides (e.g. chosen from 2'-O-methyl (2OMe), 2'-O-methoxyethyl (2MOE), 5 methyl-cytosine, phosphorodiamidate morpholino oligomer (PMO), LNA, 2'-O,4'-C-Ethylene-bridged Nucleic Acids (ENA), and/or 2' Fluoro modified nucleotides), and it hybridizes (in particular with 100% complementarity) to a predetermined target site encompassing or consisting of the slippery sequence and flanking regions within the - 1 frameshifting element (-1 FSE) region of the SARS-CoV-2 viral RNAs (i.e. nucleotides sequence 13330-13621, in particular 13370-13555).

According to a particular embodiment of the present invention, the gapmer ASO of the present invention is further characterized in that it knocks down the replication of SARS-CoV-2 in human cells with pan activity against all variants. In particular, the complete inhibition is obtained at a concentration of less than 50 nM, preferably at a concentration of less than 25 nM, more preferably at a concentration of about 5 nM.

According to a particular embodiment of the present invention, the gapmer ASO of the present invention comprises or consists of 15 to 25 hybridized nucleotides complimentary to the predetermined target site, and wherein it contains at least 5, preferably 8 to 10, contiguous deoxynucleotides in the central region that allows cleavage of SARS-CoV-2 RNA at the target site. In other words, the at least 5-deoxynucleotides central region contains the cleavage site of SARS-CoV-2 RNA at the target site.

According to a particular embodiment of the present invention, the gapmer ASO of the present invention comprises or consists of a sequence chosen from the group consisting of 5'-CCCG(T or U)TTAAAAACGAT(T or U)G(T or U)GC-3' (SEQ ID NO. 1), 5'-(T or U)CACAACTACAGCCATAACC(T or U)-3' (SEQ ID NO: 2), 5'-G(T or U)CAAAAGCCCTGTATACGAC-3' (SEQ ID NO: 3), 5'-GCACGGTGTAAGACGGGC(T or U)GC-3' (SEQ ID NO: 4), 5'-CGGAGTTGATCACAAC(T or U)ACA-3' (SEQ ID NO: 5), and 5'-CCGCAAACCCGTTTAAAAACG-3' (SEQ ID NO: 6).

According to a particular embodiment of the present invention, the gapmer ASO of the present invention hybridizes to a predetermined target site of the SARS-CoV-2 viral RNAs chosen from the group consisting of the sequence CGUUUUUAAACGGGUUUGCGG (SEQ ID NO: 7), GCACAAUCGUUUUUAAACGGG (SEQ ID NO: 8), GCAGCCCGUCUUACACCGUGC (SEQ ID: 9), or GUCGUAUACAGGGCUUUUGAC (SEQ ID NO: 10).

Another object of the present invention concerns a pharmaceutical composition comprising at least one gapmer ASO of the present invention, and a pharmaceutically acceptable excipient.

Another object of the present invention concerns a gapmer ASO or a pharmaceutical composition of the present invention, for use as a drug.

Another object of the present invention concerns a gapmer ASO or a pharmaceutical composition of the present invention, for use in the treatment of COVID-19.

### Brief description of the figures

**Figure 1****:** Secondary structures of the -1 FSE. A) SARS-CoV-2 genome with FSE. B) A possible secondary structure of the FSE derived from DMS-MaPseq data [23,26]. The -1 attenuator and slippery site (underlined) are indicated. The italic, bold and circled bases code corresponds to the three-stemmed pseudoknot elements of panel E. C) Secondary structure of the domain obtained by SHAPE-MaP RNA probing on a domain starting at nucleotide 13402 therefore lacking the upstream sequence [24]. D) Secondary structure of the domain obtained by SHAPE-MaP RNA probing on the entire viral RNA [11]. E) Three-stemmed pseudoknot that is expected to form when the ribosome sits on the slippery sequence [9,21,27]. Shaded in grey are nucleotides that would be located in the mRNA channel of the human 40S ribosomal subunit at the time of the frameshift [28]. Differences with the SARS-CoV sequence are indicated by arrows and bases in light grey (deletion: Δ). The inset indicates a possible alternative structure of the attenuator hairpin identified in panel B and C. Italic, bold letters and grey circles represent respectively sequences that form stems 1, 2 and 3 in the three-stemmed pseudoknot displayed in panel E.
**Figure 2****:** Nucleotide mutations found in variants of SARS-CoV-2 and SARS-CoV-1 compared to Wuhan-Hu-1 genome sequence (NC_045512.2). Schematic representations of the genome structure of SARS-CoV-2 and FSE region are shown on the top. The FSE RNA target indicated is highly conserved among all coronavirus genomes, making it a very promising therapeutic target.
**Figure 3****.** RNase H cleavage of SARS-CoV-2 FSE RNA inactivates frameshifting. A) Dual luciferase assay in HeLa *in vitro* translation system. B) Corresponding levels of inhibition for frameshifting stimulated by SARS-CoV-2 FSE. Frameshifting levels were estimated by monitoring the expression of Renilla and Firefly luciferase in an *in vitro* translation system from HeLa cell. The system is supplemented by translation accessory factors. C) ASO-induced cleavage of FSE RNA. RNA and ASOs were incubated in the *in vitro* translation system at 30°C and the extend of RNase H cleavage as a function of time was evaluated by RT-qPCR. RNase inhibitors were omitted. The target site for RNase H cleavage is located in between the hybridization sites of the PCR primers. Following RNase H cleavage, the reverse transcription reaction will generate a truncated reverse transcript which will not be amplified during PCR as one of the primer binding sites is absent. D) RNA cleavage induced by unmodified ASO1, ASO3, ASO4 and ASO5 as well as modified ASO1 in HeLa cell extract. Error bars are s. e. m. for triplicates.
**Figure 4****:** Activity of modified ASO1 gapmer (ASO1mod) on SARS-CoV-2 viral replication.
**Figure 5****:** *In vitro* cleavage efficiency of modified ASOs or "gapmers" on SARS-CoV-2 viral genomic RNA.
**Figure 6****:** Viral inhibition potential of different modified ASOs on Beta and Delta SARS-CoV-2 variants.
**Figure 7****:** Viral inhibition potential of ASO6mod on Beta, Delta and omicron (JN.1) variants of SARS-CoV-2, and cellular cytotoxicity profile.

### EXAMPLES

### EXAMPLE 1: MATERIALS ANS METHODS

### A- Analysis of nucleotide mutations found in SARS-CoV-2 variants and SARS-CoV-1.

List of mutated nucleotides for variants, alpha, beta, gamma, delta, lambda, mu, epsilon, eta, iota, kappa and omicron subvariants, B.1.1.529, BA.4 and BA.5 were obtained from UCSC genome browser (https://genome.ucsc.edu/index.html). For the omicron subvariants whose mutation information was not available from the UCSC browser on January 2023, sequences were obtained from GISAID (https://www.epicov.org) and aligned using Mafft program [29] and analyzed with Jalview software [30] to obtain consensus sequence of each variant. The consensus sequence was then compared to Wuhan-Hu-1 reference sequence (NC_045512.2) for identifying mutated nucleotides. The information of the sequence data used for the analysis and the cut-off values for obtaining consensus sequence is shown in the table 1 below.

**Table 1: information on GISAID sequence data and cut-off values used for mutation analysis**

| Variant name | origin | Sequence criteria | virus numbers | Cut-off value for consensus sequence |
|---|---|---|---|---|
| BA.1 | Europe | Complete High cover | 2365 | 0.75 |
| BA.2 | France | Complete High cover | 3519 | 0.75 |
| BA.3 | World | Low cover excluded | 104 | 0.75 |
| BA.2.75 | World | Complete High cover | 1204 | 0.9 |
| BA.2.75.5 | World | Complete High cover | 349 | 0.9 |
| XBB | World | Complete High cover | 160 | 0.85 |
| BQ.1 | World | Complete High cover | 328 | 0.8 |
| BQ.1.1 | World | Complete High cover | 1038 | 0.85 |
| CH.1.1 | Europe | Complete High cover | 836 | 0.85 |
| BF.7 | Europe | Complete High cover | 1964 | 0.8 |
| BA.2.3.20 | Europe | Complete High cover | 150 | 0.85 |
| BN.1 | Europe | Complete High cover | 260 | 0.85 |
| XBC.1 | Europe | Complete High cover | 114 | 0.8 |
| XBB.1.5 | Europe | Complete High cover | 260 | 0.8 |

### B- Cell culture and viruses

A549 Cl33 cell line is a gift from O. Schwartz laboratory. Vero E6 (ATCC) and Vero E6-TMPRSS2 (NIBSC, UK) cells were maintained in Dulbecco's Modified Eagle Medium (DMEM; ThermoFisher) supplemented with 10% fetal bovine serum (FBS) and 1% Penicillin/Streptomycin. A549 Cl33 cells were cultured in presence of 10 µg/mL of Blasticidin (Sigma) and Vero E6-TMPRSS2 were cultured in presence of 1 mg/mL Geneticin (ThermoFisher). Cells were maintained at 37°C in 5% CO2. All SARS-CoV-2 strains used in this study (Delta, BA.2.86, JN.1, Beta and XBB 1.5) were supplied by the National Reference Centre for Respiratory Viruses hosted by Institut Pasteur (Paris, France) and headed by Drs S. van der Werf and M.A. Rameix-Welti. Infectious stocks were produced by inoculating Vero E6-TMPRSS2 cells and collecting the cell culture media upon observation of cytopathic effect; debris were removed by centrifugation. The supernatant was then aliquoted and stored at -80°C.

### C- Synthesis of antisense oligonucleotides

ASOs were purchased from Integrated DNA Technologies (IDT). All ASOs underwent standard desalting purification after synthesis. ASOs labelled with Cy3 at the 5' end were purified by HPLC and then subjected to Na+ salt exchange to ensure that they were free from impurities and biologically compatible.

### Construction of templates for frameshifting assay and RNase H cleavage assay

The Firefly and Renilla luciferase genes were PCR-amplified from pGL4.14 and pGL4.75 plasmids (promega), respectively. The frameshift sequence of SARS-CoV2 (region 13457-13550) was prepared by primer extension using overlapping DNA oligos. The plasmid pT7CFE1-CHis (Thermo scientific) was digested using EcoRI and the backbone fragment (Pstl - Mscl) was PCR amplified. The DNA fragments were assembled using either In-Fusion Cloning kit (Takara Bio) or NEBuilder HiFi DNA Assembly kit (New England Biolabs). In-frame control plasmid for the frameshift assay contains a 30-bp linker in between the two luciferases gene. DNA primers having a part of the linker sequence were used for amplifying luciferase genes to introduce the linker. A plasmid carrying a BamHI site in between luciferase genes was constructed in a similar manner to introduce longer FSE sequence. The DNA fragment containing the region 13366 to 13554 of SARS-CoV2 was synthesized (Eurofins Genomics), PCR amplified and inserted in between the luciferase genes. The sequences of all constructs were verified by Sanger sequencing. The sequences of all the DNA primers used for preparation of the constructs are listed in table 2 below.

**Table 2**

| | DNA oligomers used in this work | |
|---|---|---|
| DNA | sequence | comments |
| RenFW | GAAAAACACGATGATAATATGGCTTCCA AGGTGTACG (SEQ ID NO : 11) | PCR primer for cloning R-luc |
| RenRev | CACCGCAAACCCGTTTAAAAACTGCTC GTTCTTCAGCACG (SEQ ID NO : 12) | PCR primer for cloning R-luc |
| PRFFW | TTTTTAAACGGGTTTGCGGTGTAAGTGC AGCCCGTCTTACACCGTGCGGCACAG (SEQ ID NO : 13) | PCR primer for cloning 13457-13550 region of FSE |
| PRFRev | GTCAAAAGCCCTGTATACGACATCAGTA CTAGTGCCTGTGCCGCACGGTGTAAGA C (SEQ ID NO : 14) | PCR primer for cloning 13457-13550 region of FSE |
| FF-Luc | GTCGTATACAGGGCTTTTGACATGGAA GATGCCAAAAACATTAAGAAGG (SEQ ID NO : 15) | PCR primer for cloning F-luc |
| FFLucRev | GCGTCGACGAGCTCCTGCAGTTATTAC ACGGCGATCTTGCCG (SEQ ID NO : 16) | PCR primer for cloning F-luc |
| pT7CFE1CHI SREV | CATATTATCATCGTGTTTTCAAAGGAA AACCAC (SEQ ID NO : 17) | PCR primer for amplifying pT7CFE1-His backbone |
| pT7CFE1CHI SFw | GGCTGGTGCTGGGGTCGGTGCTCATCT GCTCGTTCTTCAGCACG (SEQ ID NO : 18) | PCR primer for amplifying pT7CFE1-His backbone |
| In-frame Rev | GGCTGGTGCTGGGGTCGGTGCTCATCT GCTCGTTCTTCAGCACG (SEQ ID NO : 19) | PCR primer for cloning control in-frame dual-luc plasmid |
| In-frame FW | CACCGACCCCAGCACCAGCCTGAGCAT GGAAGATGCCAAAAACATTAAGAAGG (SEQ ID NO : 20) | PCR primer for cloning control in-frame dual-luc plasmid |
| In-frame Bam Rev | TTGGCATCTTCCATGGATCCCTGCTCGT TCTTCAGCACG (SEQ ID NO : 21) | PCR primer for cloning in-frame dual-luc plasmid with Bam cloning site |
| In-frame Bam-FW | TGAAGAACGAGCAGGGATCCATGGAAG ATGCCAAAAACATTAAGAAGG (SEQ ID NO : 22) | PCR primer for cloning in-frame dual-luc plasmid with Bam cloning site |
| FSnewclustF w | AGCGCGTGCTGAAGAACGAGCAGGAAT TCACAGTCTGTACCGTCTGC (SEQ ID NO : 23) | PCR primer for amplifying 13366-13554 region of FSE |
| FSnewclustR ev | CCTTCTTAATGTTTTTGGCATCTTCCAT (SEQ ID NO : 24) | PCR primer for amplifying 13366-13554 region of FSE |

### In vitro frameshifting and RNA cleavage assays

The mRNAs used for *in vitro* frameshifting assay were prepared by *in vitro* transcription using T7 RNA polymerase and purified using Nucleospin RNA spin columns (Macherey-Nagel) [31]. *In vitro* translation reactions were performed using Human *In Vitro* Protein Expression Kit (Pierce) accordingly to the instructions from the manufacturer. Typically, one reaction mixture contained 400 ng of mRNA and 10 molecular equivalents of ASO DNAs. The reaction mixture was prepared on ice in a 0.2mL thin-walled tube and translation was performed at 30°C for 2.5 hours in a thermocycler (lid temperature 60 °C). Luciferase activity was measured using the Dual-Luciferase Reporter Assay System (Promega). Fifty microliters of luciferase assay reagent were added to each sample and thoroughly mixed then transferred to a white 96-well plate (Greiner) for bioluminescence quantification using an Infinite M200 microplate reader (Tecan). Frameshifting efficiencies were calculated with the formula (*Firefly*/*Renilla*)*l*(*Firefly_{in frame}*/*Renilla_{in frame}*), where *Firefly* and *Renilla* are the respective luciferase activities and *Firefly_{in frame}* and *Renilla_{in frame}* are the luciferase activities for the control construct [32,33]. Experiments were performed in triplicate and associated error was reported as one standard deviation from the mean.

The cleavage of RNA in the presence of AS01 and ASO1mod was also assessed in the HeLa based *in vitro* translation system. After incubation at 30°C for the indicated time, an aliquot was taken and kept frozen until analysis.

### In vitro RNase H1 cleavage assay utilizing HeLa cell extract

Six microliters of HeLa cell cytoplasmic extract (Ipracell) diluted 10 times in a buffer containing 27 mM Hepes-KOH (pH 7.5), 135 mM potassium acetate, 16 mM potassium chloride, and 1.2 mM magnesium acetate, was added to a mixture of 0.5 pmols of mRNA and 5 pmols of AS DNA prepared in 4 µL of the same buffer. The mixture was incubated at 30°C for 30 min and froze in liquid nitrogen to stop the reaction. Uncleaved RNA was quantified using Luna RT-qPCR system (New England Biolabs) according to the manufacturer's instructions. The amplification was monitored either on LightCycler480 system (Roche) or on CFX Connect Real-Time System (BioRad) with following conditions: 55 °C for 10 min, 95 °C for 1 min followed by 45 cycles of 95 °C for 10 sec, 60 °C for 30 sec.

This enabled us to evaluate different gapmer ASOs in their ability to specifically bind to the targeted RNA region as well as in recruiting RNase H1 to efficiently trigger RNA cleavage.

### Inhibition Assay of SARS-CoV-2 Replication

To assess the inhibitory effect of ASOs on SARS-CoV-2 replication, a reverse transfection-based inhibition assay was performed.

### 1- Cell culture and Transfection

A549 Cl33 cells, a derivative of the human A549 cell line stably expressing the human ACE2 receptor (a kind gift from O. Schwartz), were transfected with antisense oligonucleotides (ASOs) at a final concentration of 100 nM using a reverse transfection protocol optimized in-house. Briefly, 96-well clear-bottom black plates were seeded with 20 µL of ASO solution, containing 0.6 µL of Lipofectamine 3000 (ThermoFisher) in Opti-MEM medium, per well. The plates were then completed by adding 80 µL of cell suspension (30,000 cells), following vigorous mixing. The transfection was allowed to proceed for 16 hours

### 2- Viral infection, viral load quantification and cytotoxicity assay

Following transfection, cells were inoculated with SARS-CoV-2 variants in FBS-free DMEM at various multiplicities of infection (MOls): 0.1 PFU/mL for the Delta and BA.2.86 variants, 0.2 PFU/mL for the JN.1 variant, and 0.4 PFU/mL for the Beta and XBB.1.5 variants. After a 1-hour adsorption at 37°C, the inoculum was removed, and the cells were cultured in DMEM supplemented with 2% FBS. The cells were then incubated at 37 °C for 3 or 4 days. Supernatants were collected and heat inactivated at 80°C for 20 minutes. The Luna Universal One-Step RT-qPCR Kit (New England Biolabs, USA) was used for the detection of viral genomes in the heat-inactivated samples performed through reverse transcription quantitative polymerase chain reaction (RT-qPCR). Specific primers targeting the N gene region of SARS-CoV-2 (5'-TAATCAGACAAGGAACTGATTA-3' (SEQ ID NO: 25) and 5'-CGAAGGTGTGACTTCCATG-3' (SEQ ID NO: 26)) were utilized. The cycling conditions involved an initial step at 55 °C for 10 minutes, followed by 95°C for 1 minute. Subsequently, 40 cycles were carried out with denaturation at 95°C for 10 seconds and annealing/extension at 60°C for 1 minute using a QuantStudio 6 thermocycler (Applied Biosystems, USA). The quantity of viral genomes is expressed as Ct and was normalized against the Ct values of the negative and positive controls.

In parallel, cytotoxicity was assessed using the CellTiter-Glo luminescent cell viability kit (Promega, USA). After 48 h incubation, 50 µL of CellTiter Glo reagent was added in each well and the luminescence was recorded using a luminometer (Berthold Technologies, Germany) with 0.5 sec integration time.

Raw data were normalized against appropriate negative (0%) and positive controls (100%) and are expressed in % of viral replication inhibition or % of cytotoxicity. Curve fits and IC₅₀/CC₅₀ values were obtained in Prism (GraphPad, USA) using the variable Hill slope model.

### EXAMPLE 2: RESULTS

### A- Conservation of the FSE domain of SARS-CoV-2

Coronaviruses depend on -1 programmed ribosomal frameshifting (-1 PRF) to express a polyprotein that encodes the RNA-dependent RNA polymerase [34,35]. In this FSE dependent mechanism, the ribosome slips backwards in the 5' direction by one nucleotide such that the translation proceeds in a new reading frame [36,37].

As the structure of FSE domain is conserved in SARS-CoV and SARS-CoV-2 [21], in 2020 we evaluated how this sequence has evolved during the early stage of the COVID-19 pandemic. Indeed, understanding how the FSE domain might change during adaptation to humans [38] was important for antiviral therapy development as therapeutic agents should target conserved regions of the RNA to avoid emergence of resistance. We independently analyzed 5,156 SARS-CoV-2 sequences available in GenBank and 27,153 sequences available in GISAID for sequence variation in the FSE region. We found that the sequence has remained largely unchanged during the early pandemic, with observed changes maintaining the overall architecture of the -1 PRF signals [22]. In the small percentage of GenBank and GISAID entries with changes in the FSE region relative to the reference sequence, we identified a single recurrent C to U change at position 13,536.

After a few years of evolution, the FSE region is still highly conserved (Figure 2).

### B- Preliminary tests of antisense inactivation of SARS-CoV-2 FSE RNA.

After showing the high degree of sequence conservation of SARS-CoV-2 FSE we thought of targeting this element for degradation using ASOs. We designed four antisense DNA oligonucleotides that hybridize to different regions of the FSE (Table 3). We anticipated that the ASO1 complimentary to the weakly structured slippery sequence would be the most efficient in degrading the RNA.

**Table 3: Sequences of first ASOs used against FSE**

| ASO Name | Sequence |
|---|---|
| ASO1 | CCGCAAACCCGTTTAAAAACG (SEQ ID NO: 6) |
| ASO2 | ATTGTAGATGTCAAAATGCAC (SEQ ID NO: 27) |
| ASO3 | CAGTACTAGTGCCTGTGCCGC (SEQ ID NO: 28) |
| ASO4 | CCGTTTAAAAACGATTGTGCA (SEQ ID NO: 29) |
| ASO5.1 | CCCGTTTAAAAACGATTGTGC (SEQ ID NO : 30) |
| ASO1 mod | [2OMeC]*[2OMeC]*[2OMeG]*[2OMeC]*[2OMeA]*A*A*C*C*C*G*T*T*T*A*A* [2OMeA]*[2OMeA]*[2OMeA]*[2OMeC]*[2OMeG] (SEQ ID NO : 31) |

First, the efficiency of each ASO to inhibit -1 frameshifting was measured using a dual luciferase assay (Figure 3A). All ASOs were 21 nucleotides in length (ASO4 has 14 nucleotides hybridizing to the target model RNA). All tested ASOs induced inhibition of -1 frameshifting although to different extent (Figure 3B). ASO1 and ASO4 designed to bind to the slippery sequence/linker region had the strongest activity. The results indicate that the slippery sequence and linker region of FSE is an excellent target site for ASOs.

Next, we evaluated the capacity of ASO1 to degrade *in vitro* the SARS-CoV-2 FSE RNA by recruiting RNAse H1. ASO1 cleaved more than 80% of the RNA within 30 minutes of incubation in an *in vitro* translation system in conditions of active protein synthesis (Figure 3C). The gapmer ASO1mod, which contains phosphorothioate linkages and a central sequence flanked by five 2'OMe modified nucleotides on the 5' and 3' sides induced a similar rapid decay of FSE RNA. Extending the assay to other unmodified ASOs showed that ASO4 and ASO5 that originate from ASO1 were the most active (Figure 3D). ASO4 is shifted by 7 nucleotides in the 5' direction compared to ASO1. ASO5 results from a microwalk of ASO4 with a single nucleotide shift in the 5' direction.

Before to engage in a deeper study with different ASOs targeting FSE we first validated the activity of ASO1mod in human lung cells A549-ACE2 (Figure 4). Results showed a very strong *in cellulo* activity with no cytotoxic effect.

### C- Antisense inactivation of SARS-CoV-2 FSE RNA.

We next performed a deeper study including more advanced ASOs in a RNase H1-dependent cleavage assay in a HeLa cell extract. Modified versions of antisense oligonucleotides targeting the FSE RNA domain have been developed for *in cellulo* and potentially *in vivo* experiments. In addition to the universality of sequence conservation of the target sequence additional parameters were fine tuned for choosing the sequence: the melting temperature of the DNA-RNA hybrid (adjusted with the composition of modifications in the wing of the gapmers), the bioinformatic off-target assessments, RNP-Map data for the interaction of viral RNA with protein N, DMS-MaP, SHAPE-MaP, COMRADES data for target site accessibility within multiple secondary structures [11,23,24,25]. All the sequences and modifications of the ASOs used are listed in Table 4. These modifications were introduced mainly to enhance their resistance to cellular endonucleases and to modulate their affinities for the FSE RNA target [39]. As shown in Table 4, the main modifications are located on the 2'OH group of the ribose and also consist in the replacement of phosphoester bonds by phosphorothiate (PS) bonds on the phosphate backbone of the oligonucleotides, thus generating products with a racemic mixture of R or S conformation.

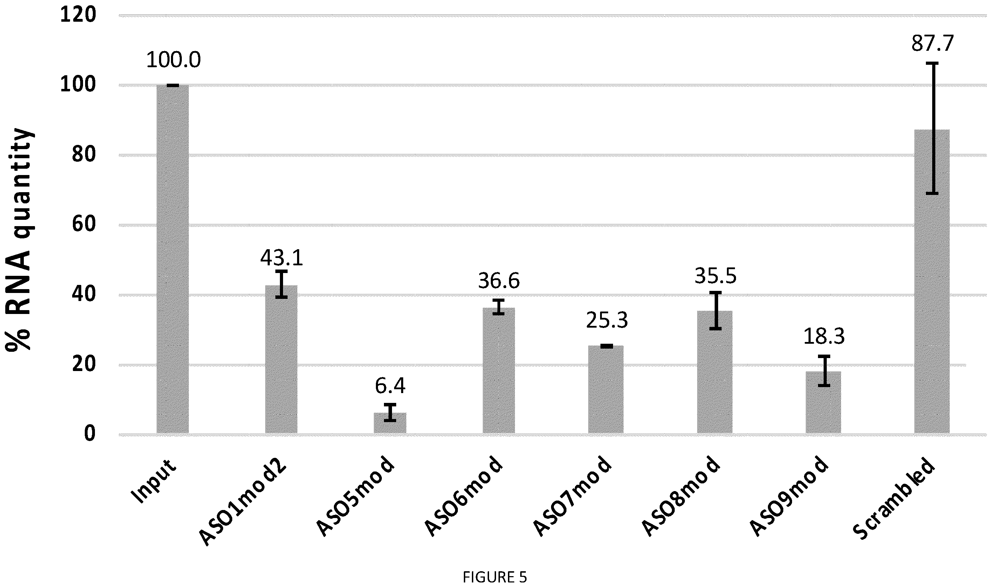

Whereas scrambled (negative control: oligonucleotide with a random control sequence having the same base composition as ASO1 with 3T/U, 8A,7C,3G but that does not hybridize to viral RNA) exerted no cutting activity, we showed that the modified ASO1, ASO5, ASO6, ASO7, ASO8 and ASO9 specifically cut viral RNA in the following order of decreasing efficiency:
ASO5mod>ASO9mod>ASO7mod>ASO6mod =ASO8mod>ASO1mod2 (Figure 5).

**Table 4: Sequences of all modified ASOs used in this project**

| **ASO Name** | **Sequence** |
|---|---|
| ASO1mod2 (SEQ ID NO : 32) | 5'-/52MOErC/*/i2MOErC/*mG*mC*mA*A*A*C*C*C*G*T*T*T*A*A*mA*mA*mA*/i2MOErC/*/32MOErG/-3' |
| ASO5mod (SEQ ID NO : 33) | 5'-/52MOErC/*/i2MOErC/*/i2MOErC/*mG*mU*T*T*A*A*A*A*A*C*G*A*T*mU*mG*mU*mG*/32MOErC/-3' |
| ASO5modCy3 (SEQ ID NO : 34) | /5Cy3//i2MOErC/*/i2MOErC/*/i2MOErC/*mG*mU*T*T*A*A*A*A*A*C*G*A*T*mU*mG*mU*mG*/32MOErC/ |
| ASO6mod (SEQ ID NO : 35) | 5'-mG*mC*mA*mC*mG*G*T*G*T*A*A*G*A*C*G*G*mG*mC*mU*mG*mC-3' |
| ASO7mod (SEQ ID NO : 36) | 5'-mG*mU*mC*mA*mA*A*A*G*C*C*C*T*G*T*A*T*mA*mC*mG*mA*/32MOErC/-3' |
| ASOSmod (SEQ ID NO : 37) | 5'-/52MOErC/*mG*mG*mA*mG*T*T*G*A*T*C*A*C*A*A*C*mU*mA*mC*mA*/32MOErG/-3' |
| ASOSmod (SEQ ID NO : 38) | 5'-mU*mC*mA*mC*mA*A*C*T*A*C*A*G*C*C*A*T*mA*mA*mC*mC*mU-3' |
| ASO10mod (SEQ ID NO : 39) | 5'-mC*mC*mC*mA*mC*A*G*G*G*T*C*A*T*T*A*mG*mC*mA*mC*mA-3' |
| ASO11mod (SEQ ID NO : 40) | 5'-mC*mG*mU*mC*mC*T*T*T*T*C*T*T*G*G*A*A*mG*mC*mG*mA*mC-3' |
| ASO12mod (SEQ ID NO : 41) | 5'-/52MOErA/*/i2MOErG/*/i2MOErG/*mA*mA*T*T*T*A*G*C*A*A*A*A*C*mC*mA*mG*mC*/32MOErT/-3' |
| Scrambled (SEQ ID NO : 42) | 5'-/52MOErT/*/i2MOErA/*mC*mU*mC*C*A*A*T*C*G*G*A*A*C*A*mA*mC*mG*/i2MOErC/*/32MOErA/-3' |
| Scrambled Cy3 (SEQ ID NO : 43) | 5Cy3//i2MOErT/*/i2MOErA/*mC*mU*mC*C*A*A*T*C*G*G*A*A*C*A*mA*mC*mG*/i2MOErC/*/32MOErA/ |

| | |
|---|---|
| "*" indicates phosphorothioate bonds; "m" indicates 2'-O-methyl base; "/52MOEr_/", "/i2MOEr_/", and "/32MOEr_/" indicates 2'-O-methoxyethyl base at 5' end, internally, and 3' end, respectively. For example, for MOErC there is a 5-methyl modification on 2'-O-methoxyethyl C. | |

On the basis of our *in vitro* experiments, we then decided to assess the potential for inhibiting SARS-CoV-2 viral replication in a cell-based assay, using all the modified ASOs found to be active in the RNase-H1-mediated viral genomic RNA cleavage assay.

### D- In vitro viral replication assay validates the pan-antiviral activity of ASOs

In order to assess the pan-antiviral activity of modified ASOs or "gapmers" on living human cells, we first had to develop robust antiviral assays under BSL3 laboratory conditions with all SARS-CoV-2 variants. Different variants have been shown to have different cellular replication kinetics. In parallel, we had to develop pre- and post-viral infection cytotoxicity assays: (i) because ASO modifications complemented by transfection agents can induce cellular cytotoxicity, (ii) they can also alter RNA target specificity, leading to an increased "off-target" cytotoxicity. The inhibition potency of each ASO was then determined using its CC₅₀/IC₅₀ therapeutic index, which corresponds to the ASO concentration required to reduce cell viability by 50% (CC50) divided by the minimum concentration for inhibiting 50% of virus replication (IC50). In addition, different assays had to be adapted with the same modified ASOs, by varying transfection kinetics at 64 h or 96 h, to assess the time dependent antiviral inhibition effect.

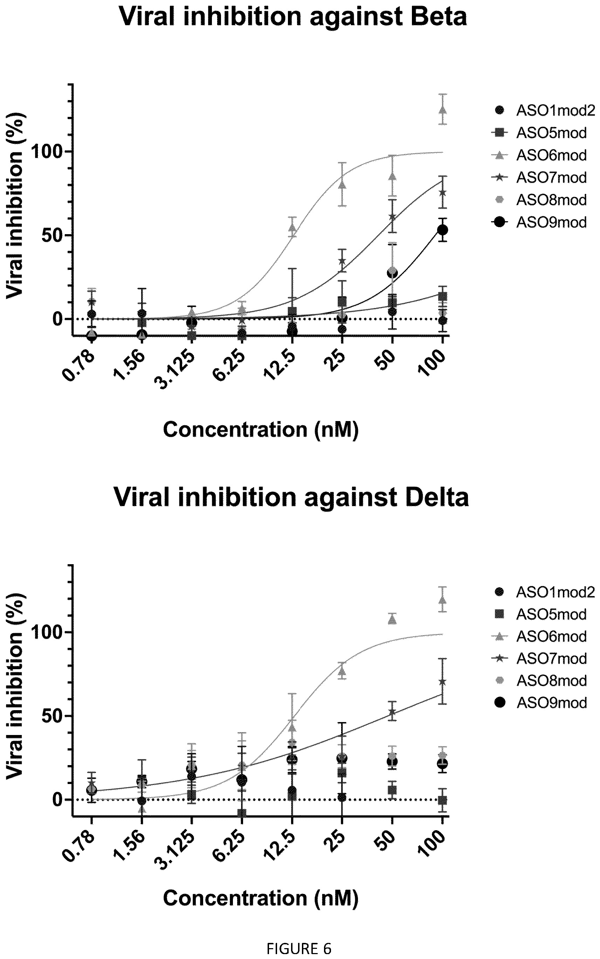

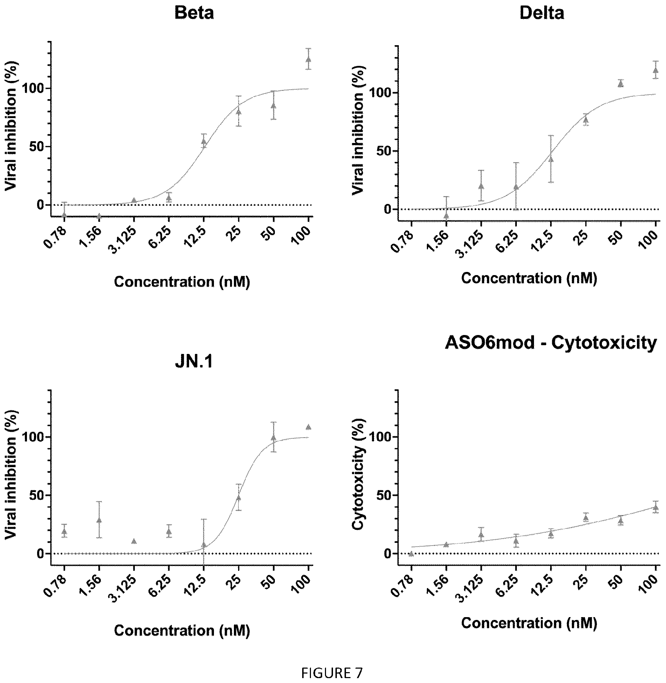

Briefly, the antiviral assay we had to optimize for several months is as follows: human lung cell lines (A549) were infected with the authentic Beta, Delta, BA.2.86, XBB.1.5 and JN.1 variants of SARS-CoV-2 under BSL-3 conditions, using human cells pre-transfected with gapmeric ASOs. We then determined the potential antiviral inhibitory effect of ASOs on virus replication by measuring by RT-qPCR viral RNA levels following infection, and determined in parallel cytotoxicity using a luminescence cell viability assay (CellTiter-Glo, Promega) to calculate their therapeutic indices (CC₅₀/IC₅₀). As shown in Table 3 and Figures 6-7, we showed that the two gapmers ASO6mod and ASO7mod exhibit potent pan-antiviral activity, with IC₅₀ values in the nanomolar range, values that remain virtually unchanged regardless of whether Beta, Delta, or Omicron variants (such as BA.2.86, XBB.1.5, or JN.1) are used. These results validate the main strength of our antiviral therapeutic strategy using modified antisense oligonucleotides, known as "gapmers", and show for the first time pan-antiviral activity against all SARS-CoV-2 variants. Furthermore, as the FSE target region of genomic RNA is highly conserved in coronaviruses, it is highly likely that this antiviral activity of ASO6mod and ASO7mod can be preserved in all other coronaviruses, such as SARS-CoV-1, HCoV-229E and HCoV-OC43.

**Table 5: Determination of the antiviral activity of ASOs**

| ASO Name and SEQ ID NO | IC₅₀ against Beta (nM) | IC₅₀ against Delta (nM) | IC₅₀ against BA.2.86 (nM) | IC₅₀ against XBB.1.5 (nM) | IC₅₀ against JN.1 (nM) | Cytotoxicity CC₅₀ (nM) |
|---|---|---|---|---|---|---|
| ASO1mod2 (SEQ ID NO: 32) | >100 | >100 | >100 | >100 | N.D | >100 |
| ASO5mod (SEQ ID NO: 33) | >100 | >100 | >100 | >100 | N.D | >100 |
| ASO5modCy3 (SEQ ID NO: 34) | >100 | >100 | >100 | >100 | N.D | >100 |
| ASO6mod (SEQ ID NO: 35) | 12.76 | 12.92 | 5.84 | 12.76 | 24.91 | >100 |
| ASO7mod (SEQ ID NO: 36) | 41.19 | 46.99 | 42.16 | 41.19 | N.D | >100 |
| ASO8mod (SEQ ID NO: 37) | >100 | >100 | >100 | >100 | N.D | >100 |
| ASO9mod (SEQ ID NO: 38) | 90.14 | >100 | >100 | 90.14 | N.D | >100 |

Note that ASO1 mod had strong activity in Figure 4 whereas ASO1mod2 showed lower activity here. This is due to adjustments in the protocol that took into account longer replication rates for some variants (see methods).

### List of references

(1) Lim, K. R. Q.; Yokota, T. Invention and Early History of Gapmers. Methods Mol Biol 2020, 2176, 3-19. https://doi.org/10.1007/978-1-0716-0771-8_1.
(2) Liang, X.-H.; Sun, H.; Nichols, J. G.; Crooke, S. T. RNase H1-Dependent Antisense Oligonucleotides Are Robustly Active in Directing RNA Cleavage in Both the Cytoplasm and the Nucleus. Molecular Therapy 2017, 25 (9), 2075-2092. https://doi.org/10.1016/j.ymthe.2017.06.002.
(3) Khvorova, A.; Watts, J. K. The Chemical Evolution of Oligonucleotide Therapies of Clinical Utility. Nat. Biotechnol. 2017, 35 (3), 238-248. https://doi.org/10.1038/nbt.3765.
(4) Yu, A.-M.; Choi, Y. H.; Tu, M.-J. RNA Drugs and RNA Targets for Small Molecules: Principles, Progress, and Challenges. Pharmacol. Rev. 2020, 72 (4), 862-898. https://doi.org/10.1124/pr. 120.019554.
(5) Roberts, T. C.; Langer, R.; Wood, M. J. A. Advances in Oligonucleotide Drug Delivery. Nature Reviews Drug Discovery 2020, 19 (10), 673-694. https://doi.org/10.1038/s41573-020-0075-7.
(6) Egli, M., and Manoharan, M. (2023). Chemistry, structure and function of approved oligonucleotide therapeutics. Nucleic Acids Res. 51, 2529-2573. https://doi.org/10.1093/nar/gkad067.- Mullard, A. (2024). 2023 FDA approvals. Nat. Rev. Drug Discov. 23.
(7) Mullard, A. 2023 FDA Approvals. Nature Reviews Drug Discovery 2024, 23 (2), 88-95. https://doi.org/10.1038/d41573-024-00001-x.
(8) Verdin, P. FDA New Drug Approvals in Q2 2024. Nature Reviews Drug Discovery 2024, 23 (8), 573-573. https://doi.org/10.1038/d41573-024-00118-z.
(9) Zhang, K.; Zheludev, I. N.; Hagey, R. J.; Haslecker, R.; Hou, Y. J.; Kretsch, R.; Pintilie, G. D.; Rangan, R.; Kladwang, W.; Li, S.; Wu, M. T.-P.; Pham, E. A.; Bernardin-Souibgui, C.; Baric, R. S.; Sheahan, T. P.; D'Souza, V.; Glenn, J. S.; Chiu, W.; Das, R. Cryo-EM and Antisense Targeting of the 28-KDa Frameshift Stimulation Element from the SARS-CoV-2 RNA Genome. Nat. Struct. Mol. Biol. 2021, 28 (9), 747-754. https://doi.org/10.1038/s41594-021-00653-y.
(10) Zhu, C.; Lee, J. Y.; Woo, J. Z.; Xu, L.; Nguyenla, X.; Yamashiro, L. H.; Ji, F.; Biering, S. B.; Van Dis, E.; Gonzalez, F.; Fox, D.; Wehri, E.; Rustagi, A.; Pinsky, B. A.; Schaletzky, J.; Blish, C. A.; Chiu, C.; Harris, E.; Sadreyev, R. I.; Stanley, S.; Kauppinen, S.; Rouskin, S.; Näär, A. M. An Intranasal ASO Therapeutic Targeting SARS-CoV-2. Nat. Commun. 2022, 13, 4503. https://doi.org/10.1038/s41467-022-32216-0. (WO 2022/031410)
(11) Huston, N. C.; Wan, H.; Strine, M. S.; Tavares, R. de C. A.; Wilen, C. B.; Pyle, A. M. Comprehensive in Vivo Secondary Structure of the SARS-CoV-2 Genome Reveals Novel Regulatory Motifs and Mechanisms. Mol. Cell 2021, 81 (3), 584-598.e5. https://doi.org/10.1016/j.molcel.2020.12.041.
(12) Li, Y.; Garcia, G.; Arumugaswami, V.; Guo, F. Structure-Based Design of Antisense Oligonucleotides That Inhibit SARS-CoV-2 Replication. bioRxiv 2021, 2021.08.23.457434. https://doi.org/10.1101/2021.08.23.457434. (WO 2023/009396)
(13) Lulla, V.; Wandel, M. P.; Bandyra, K. J.; Ulferts, R.; Wu, M.; Dendooven, T.; Yang, X.; Doyle, N.; Oerum, S.; Beale, R.; O'Rourke, S. M.; Randow, F.; Maier, H. J.; Scott, W.; Ding, Y.; Firth, A. E.; Bloznelyte, K.; Luisi, B. F. Targeting the Conserved Stem Loop 2 Motif in the SARS-CoV-2 Genome. J. Virol. 2021. https://doi.org/10.1128/JVI.00663-21.
(14) Qiao, Y.; Wotring, J.W.; Zhang, C.J.; Jiang, X.; Xiao, L.; Watt, A.; Gattis, D.; Scandalis, E.; Freier, S.; Zheng, Y.; Pretto, C. D.; Ellison, S. J.; Szayze, E. E.; Guo, S.; Sexton, J. Z.; Chinnaiyan, A. M. Antisense oligonucleotides to therapeutically target SARS-CoV-2 infection. PLOS ONE . 2023, 18, e0281281. https://doi.org/10.1371/journal.pone.0281281.
(15) Yamasaki, M.; Saso, W.; Yamamoto, T.; Sato, M.; Takagi, H.; Hasegawa, T.; Kozakura, Y.; Yokoi, H.; Ohashi, H.; Tsuchimoto, K.; Hashimoto, R.; Fukushi, S.; Uda, A.; Muramatsu, M.; Takayama, K.; Maeda, K.; Takahashi, Y.; Nagase, T.; Watashi, K. Anti-SARS-CoV-2 gapmer antisense oligonucleotides targeting the main protease region of viral RNA. Antiviral Res. 2024, 230, 105992. https://doi.org/10.1016/j.antiviral.2024.105992.
(16) Park S, Kim SH, Dezhbord M, Lee E-H, Jeon Y, Jung D, Gu SH, Yu C, Lee SH, Kim SC and Kim K-H (2023) Cell-permeable peptide nucleic acid antisense oligonucleotide platform targeting human betacoronaviruses. Front. Microbiol 2023, 14:1258091. doi: 10.3389/fmicb.2023.1258091
(17) Cao, C.; Cai, Z.; Xiao, X.; Rao, J.; Chen, J.; Hu, N.; Yang, M.; Xing, X.; Wang, Y.; Li, M.; Zhou, B.; Wang, X.; Wang, J.; Xue, Y. The Architecture of the SARS-CoV-2 RNA Genome inside Virion. Nat. Commun. 2021, 12 (1), 3917. https://doi.org/10.1038/s41467-021-22785-x.
(18) Idris, A.; Davis, A.; Supramaniam, A.; Acharya, D.; Kelly, G.; Tayyar, Y.; West, N.; Zhang, P.; McMillan, C. L. D.; Soemardy, C.; Ray, R.; O'Meally, D.; Scott, T. A.; McMillan, N. A. J.; Morris, K. V. A SARS-CoV-2 Targeted SiRNA-Nanoparticle Therapy for COVID-19. Mol. Ther. 2021, 29 (7), 2219-2226. https://doi.org/10.1016/j.ymthe.2021.05.004.
(19) Idris, A.; Supramaniam, A.; Tayyar, Y.; Kelly, G.; McMillan, C. L. D.; Morris, K. V. An intranasally delivered ultra-conserved siRNA prophylactically represses SARS-CoV-2 infection in the lung and nasal cavity. Antiviral Res. 2024, 222, 105815. https://doi.org/10.1016/j.antiviral.2024.105815.
(20) Abbott, T. R.; Dhamdhere, G.; Liu, Y.; Lin, X.; Goudy, L.; Zeng, L.; Chemparathy, A.; Chmura, S.; Heaton, N. S.; Debs, R.; Pande, T.; Endy, D.; La Russa, M. F.; Lewis, D. B.; Qi, L. S. Development of CRISPR as an Antiviral Strategy to Combat SARS-CoV-2 and Influenza. Cell 2020, 181 (4), 865-876.e12. https://doi.org/10.1016/j.cell.2020.04.020.
(21) Kelly, J. A.; Olson, A. N.; Neupane, K.; Munshi, S.; San Emeterio, J.; Pollack, L.; Woodside, M. T.; Dinman, J. D. Structural and Functional Conservation of the Programmed -1 Ribosomal Frameshift Signal of SARS Coronavirus 2 (SARS-CoV-2). J. Biol. Chem. 2020, 295 (31), 10741-10748. https://doi.org/10.1074/jbc.AC120.013449.
(22) Fourmy, D.; Yoshizawa, S. A Cytosine-to-Uracil Change within the Programmed -1 Ribosomal Frameshift Signal of SARS-CoV-2 Results in Structural Similarities with the MERS-CoV Signal. bioRxiv 2020, 2020.06.26.174193. https://doi.org/10.1101/2020.06.26.174193.
(23) Lan, T. C. T.; Allan, M. F.; Malsick, L. E.; Woo, J. Z.; Zhu, C.; Zhang, F.; Khandwala, S.; Nyeo, S. S. Y.; Sun, Y.; Guo, J. U.; Bathe, M.; Näär, A.; Griffiths, A.; Rouskin, S. Secondary Structural Ensembles of the SARS-CoV-2 RNA Genome in Infected Cells. Nat Commun 2022, 13 (1), 1128. https://doi.org/10.1038/s41467-022-28603-2.
(24) Iserman, C.; Roden, C. A.; Boerneke, M. A.; Sealfon, R. S. G.; McLaughlin, G. A.; Jungreis, I.; Fritch, E. J.; Hou, Y. J.; Ekena, J.; Weidmann, C. A.; Theesfeld, C. L.; Kellis, M.; Troyanskaya, O. G.; Baric, R. S.; Sheahan, T. P.; Weeks, K. M.; Gladfelter, A. S. Genomic RNA Elements Drive Phase Separation of the SARS-CoV-2 Nucleocapsid. Molecular Cell 2020, 80 (6), 1078-1091.e6. https://doi.org/10.1016/j.molcel.2020.11.041.
(25) Ziv, O.; Price, J.; Shalamova, L.; Kamenova, T.; Goodfellow, I.; Weber, F.; Miska, E. A. The Short- and Long-Range RNA-RNA Interactome of SARS-CoV-2. Mol Cell 2020. https://doi.org/10.1016/j.molcel.2020.11.004.
(26) Lan, T. C. T.; Allan, M. F.; Malsick, L. E.; Khandwala, S.; Nyeo, S. S. Y.; Bathe, M.; Griffiths, A.; Rouskin, S. Structure of the Full SARS-CoV-2 RNA Genome in Infected Cells. bioRxiv 2020, 2020.06.29.178343. https://doi.org/10.1101/2020.06.29.178343.
(27) Bhatt, P.R.; Scaiola, A.; Loughran, G.; Leibundgut, M.; Kratzel, A.; Meurs, R.; Dreos, R.; O'Connor, K.M.; McMillan, A.; Bode, J.W.; Thiel, V.; Gatfield, D.; Atkins, J. F.; Ban, N. Structural basis of ribosomal frameshifting during translation of the SARS-CoV-2 RNA genome. Science 2021, 372, 1306-1313. https://doi.org/10.1126/science.abf3546.
(28) Brito Querido, J.; Sokabe, M.; Kraatz, S.; Gordiyenko, Y.; Skehel, J. M.; Fraser, C. S.; Ramakrishnan, V. Structure of a Human 48S Translational Initiation Complex. Science 2020, 369 (6508), 1220-1227. https://doi.org/10.1126/science.aba4904.
(29) Katoh, K.; Standley, D. M. MAFFT Multiple Sequence Alignment Software Version 7: Improvements in Performance and Usability. Molecular Biology and Evolution 2013, 30 (4), 772-780. https://doi.org/10.1093/molbev/mst010.
(30) Waterhouse, A. M.; Procter, J. B.; Martin, D. M. A.; Clamp, M.; Barton, G. J. Jalview Version 2-a Multiple Sequence Alignment Editor and Analysis Workbench. Bioinformatics 2009, 25 (9), 1189-1191. https://doi.org/10.1093/bioinformatics/btp033.
(31) Okuda, M.; Fourmy, D.; Yoshizawa, S. Use of Baby Spinach and Broccoli for Imaging of Structured Cellular RNAs. Nucleic Acids Res. 2017, 45 (3), 1404-1415. https://doi.org/10.1093/nar/gkw794.
(32) Grentzmann, G.; Ingram, J. A.; Kelly, P. J.; Gesteland, R. F.; Atkins, J. F. A Dual-Luciferase Reporter System for Studying Recoding Signals. RNA 1998, 4, 479-486.
(33) Mazauric, M.-H.; Leroy, J.-L.; Visscher, K.; Yoshizawa, S.; Fourmy, D. Footprinting Analysis of BWYV Pseudoknot-Ribosome Complexes. RNA 2009, 15, 1775-1786. https://doi.org/10.1261/rna. 1385409.
(34) Brierley, I.; Boursnell, M. E.; Binns, M. M.; Bilimoria, B.; Blok, V. C.; Brown, T. D.; Inglis, S. C. An Efficient Ribosomal Frame-Shifting Signal in the Polymerase-Encoding Region of the Coronavirus IBV. EMBO J. 1987, 6 (12), 3779-3785.
(35) Irigoyen, N.; Firth, A. E.; Jones, J. D.; Chung, B. Y.-W.; Siddell, S. G.; Brierley, I. High-Resolution Analysis of Coronavirus Gene Expression by RNA Sequencing and Ribosome Profiling. PLoS Pathog. 2016, 12 (2), e1005473. https://doi.org/10.1371/journal.ppat.1005473.
(36) Dinman, J. D. Mechanisms and Implications of Programmed Translational Frameshifting. Wiley Interdiscip Rev RNA 2012, 3 (5), 661-673. https://doi.org/10.1002/wrna.1126.
(37) Atkins, J. F.; Loughran, G.; Bhatt, P. R.; Firth, A. E.; Baranov, P. V. Ribosomal Frameshifting and Transcriptional Slippage: From Genetic Steganography and Cryptography to Adventitious Use. Nucleic Acids Res. 2016, 44 (15), 7007-7078. https://doi.org/10.1093/nar/gkw530.
(38) van Dorp, L.; Acman, M.; Richard, D.; Shaw, L. P.; Ford, C. E.; Ormond, L.; Owen, C. J.; Pang, J.; Tan, C. C. S.; Boshier, F. A. T.; Ortiz, A. T.; Balloux, F. Emergence of Genomic Diversity and Recurrent Mutations in SARS-CoV-2. Infect. Genet. Evol. 2020, 83, 104351. https://doi.org/10.1016/j.meegid.2020.104351.
(39) Crooke, S. T.; Baker, B. F.; Crooke, R. M.; Liang, X.-H. Antisense Technology: An Overview and Prospectus. Nat Rev Drug Discov 2021, 20 (6), 427-453. https://doi.org/10.1038/s41573-021-00162-z.

## Claims

1. Gapmer antisense oligonucleotide (ASO) targeting SARS-CoV-2 viral RNAs, **characterized in that** it comprises phosphorothioate linkages and a central sequence flanked by modified nucleotides on the 5' and 3' sides, and it hybridizes to a predetermined target site encompassing the slippery sequence and flanking regions within the -1 frameshifting element (-1 FSE) region of the SARS-CoV-2 viral RNAs;
wherein the target site ranges from nucleotides 13330 to 13621; and
wherein the modified nucleotides are chosen from 2'-O-methyl (2OMe), 2'-O-methoxyethyl (2MOE), 5 methyl-cytosine, phosphorodiamidate morpholino oligomer (PMO), LNA, 2'-O,4'-C-Ethylene-bridged Nucleic Acids (ENA), and/or 2' Fluoro modified nucleotides.

2. Gapmer ASO according to claim 1, **characterized in that** it knocks down the replication of SARS-CoV-2 in human cells with pan activity against all variants.

3. Gapmer ASO according to claim 1 or 2, wherein the gapmer ASO comprises 15 to 25 hybridized nucleotides complimentary to the predetermined target site, and wherein at least 5, preferably 8 to 10, contiguous deoxynucleotides in the central region comprise the cleavage site of SARS-CoV-2 RNA at the target site.

4. Gapmer ASO according to any of claims 1 to 3, wherein the gapmer ASO comprises a sequence chosen from the group consisting of 5'-CCCG(T or U)TTAAAAACGAT(T or U)G(T or U)GC-3' (SEQ ID NO: 1), 5'-(T or U)CACAACTACAGCCATAACC(T or U)-3' (SEQ ID NO: 2), 5'-G(T or U)CAAAAGCCCTGTATACGAC-3' (SEQ ID NO: 3), 5'-GCACGGTGTAAGACGGGCUGC-3' (SEQ ID NO: 4), 5'-CGGAGTTGATCACAACUACA-3' (SEQ ID NO: 5), and 5'-CCGCAAACCCGTTTAAAAACG-3' (SEQ ID NO: 6).

5. Gapmer ASO according to any of claims 1 to 4, wherein the gapmer ASO hybridizes to a predetermined target site of the SARS-CoV-2 viral RNAs chosen from the group consisting of the sequence CGUUUUUAAACGGGUUUGCGG (SEQ ID NO: 7), GCACAAUCGUUUUUAAACGGG (SEQ ID NO: 8), GCAGCCCGUCUUACACCGUGC (SEQ ID: 9), or GUCGUAUACAGGGCUUUUGAC (SEQ ID NO: 10).

6. Pharmaceutical composition comprising at least one gapmer ASO according to any one of claims 1 to 5, and a pharmaceutically acceptable excipient.

7. Gapmer ASO according to any one of claims 1 to 5 or pharmaceutical composition according to claim 6, for use as a drug.

8. Gapmer ASO according to any one of claims 1 to 5 or pharmaceutical composition according to claim 6, for use in the treatment of COVID-19.
